# EUROPEAN PATENT SPECIFICATION

(11) **EP 1 643 250 B1**
(45) Date of publication and mention of the grant of the patent: **22.02.2012**
(21) Application number: 04747013.3
(22) Date of filing: 05.07.2004
(51) Int. Cl.: G01N 33/53, G01N 37/00, C12M 1/00, C12Q 1/68

(54) **BIOCHEMICAL REACTION SYSTEM, BIOCHEMICAL REACTION SUBSTRATE, PROCESS FOR PRODUCING HYBRIDIZATION SUBSTRATE AND HYBRIDIZATION METHOD**
BIOCHEMISCHES REAKTIONSSYSTEM, BIOCHEMISCHES REAKTIONSSUBSTRAT, VERFAHREN ZUR HERSTELLUNG EINES HYBRIDISIERUNGSSUBSTRATS UND HYBRIDISIERUNGSVERFAHREN
SYSTEME DE REACTION BIOCHIMIQUE, SUBSTRAT POUR REACTION BIOCHIMIQUE, PROCEDE D'OBTENTION D'UN SUBSTRAT D'HYBRIDATION ET PROCEDE D'HYBRIDATION

(30) Priority: 07.07.2003 JP 2003193064
(43) Date of publication of application: 05.04.2006
(73) Proprietor: Sony Corporation, Tokyo 141-0001 (JP)
(72) Inventor: NAKAO, Isamu SONY CORPORATION, Tokyo 1410001 (JP); MAMINE, Takayoshi SONY CORPORATION, Tokyo 1410001 (JP); YAMAMOTO, Masanobu SONY CORPORATION, Tokyo 1410001 (JP); TAKEDA, Minoru SONY CORPORATION, Tokyo 1410001 (JP); FURUKI, Motohiro SONY CORPORATION, Tokyo 1410001 (JP)
(74) Representative: Horner, David Richard
(86) International application number: PCT/JP2004/009544
(87) International publication number: WO 2005/003770

(56) References cited:
- EP-A- 1 146 331
- JP-A- 2001 330 608
- JP-A- 2003 149 238
- JP-A- 2003 185 656
- JP-A- 2003 202 343
- JP-A- 2004 132 720
- US-A1- 2003 049 667

## Description

### Technical Field

The present invention relates to a biochemical reaction apparatus that provides biochemical reaction with the use of a substrate, a substrate for biochemical reaction (will also be referred to as "bioassay substrate" hereinbelow) such as DNA chip or the like, a method of hybridizing a nucleotide chain, and a method of producing a substrate for hybridization in which the nucleotide chain for a probe is fixed.

This application claims the priority of the Japanese Patent Application published as JP2005-030783 filed in the Japanese Patent Office on July 7, 2003.

### Background Art

These days, a substrate for biochemical reaction, called "DNA chip" or "DNA microarray" (will generically be referred to as "DNA chip" hereunder) in which a predetermined DNA (total length or part) is micro-arrayed with the microarray technology is used for analysis of mutation in genes, SNPs (single nucleotide polymorphisms), frequency of gene expression, etc. Such substrates for biochemical reaction have started being utilized in many fields such as drug discovery, clinical diagnosis, pharmacogenomics, legal medicine, etc.

In the DNA analysis using the DNA chip, mRNA (messenger RNA) extracted from a cell, tissue or the like is PCR-amplified while having a fluorescent probe-use dNTP integrated thereinto by reverse transcript PCR (Polymerase Chain Reaction) or the like to generate a sample-use DNA and the sample-use DNA is dripped onto a probe-use DNA solid-phased (fixed) on the DNA chip, to thereby hybridize the probe-use and sample-use DNAs. Then, a fluorescent marker is inserted into the double helix and fluorescence is measured using a predetermined detector. With these operations, it is determined whether the sample-use and probe-use DNAs are identical in base sequence to each other.

The Japanese Patent Application published as JP 2001-238674 discloses a hybridization speed-up technology based on the fact that DNA is negative-charged and in which a positive electrode is provided near a fixed probe-use DNA to move a drifting sample-use DNA toward the probe-use DNA, thereby speeding up the hybridization.

However, since single-strand DNA does not form any normal chain but a random coil in a solution, it is a steric hindrance to combination of probe-use and sample-use DNAs and therefore it is difficult to hybridize the DNAs at a high speed. Even if the drifting sample-use DNA is moved toward the probe-use DNA under the influence of an electric field, the steric hindrance will not be changed and hence any higher-speed hybridization is difficult.

EP1146 331 describes a gene detecting chip comprising a plurality of PIN electrodes for immobilizing PCR products, oligonucleotides, mRNA and cDNA.

US 2003/0049667 Al describes an apparatus for hybridization reaction which comprises a holder for loading a solid support and an electric field generator comprising a power controller and a first electrode plate and a second electrode plate respectively placed below or above the holder for generating a direction-convertible electric field to at least cover the solid support.

### Disclosure of the Invention

Accordingly, the present invention has an object to overcome the above-mentioned drawbacks of the related art by providing a biochemical reaction apparatus capable of hybridizing DNAs at a high speed.

The present invention has another object to provide a biochemical reaction substrate capable of high-speed hybridization and having a simpler configuration.

The present invention has still another object to provide a method of producing a biochemical reaction substrate capable of high-speed hybridization and having a simpler configuration.

The present invention has yet another object to provide a hybridizing method capable of easy, higher-speed hybridization.

The present invention is limited in the appended claims.

These objects and other objects, features and advantages of the present invention will become more apparent from the following detailed description of the best mode for carrying out the present invention when taken in conjunction with the accompanying drawings.

### Brief Description of the Drawings

FIG. 1 is a plan view of a bioassay substrate according to the present invention.
FIG. 2 is a sectional view of the bioassay substrate according to the present invention.
FIG. 3 shows steps of forming the bioassay substrate.
FIG. 4 shows silane molecules each having a to-be-modified OH group on the bottom of a well.
FIG. 5 shows a probe-use DNA combined with the well bottom.
FIG. 6 explains control on dripping of a solution onto the bioassay substrate.
FIG. 7 explains a method of applying an AC electric field to the bioassay substrate.
FIG. 8 is a block diagram of a DNA analyzer for analysis of DNA using the bioassay substrate according to the present invention.

### Best Mode for Carrying Out the Invention

The present invention will be described in detail below concerning a DNA analyzing bioassay substrate and a bioassay method of DNA analysis using the bioassay substrate as embodiments thereof with reference to the accompanying drawings.

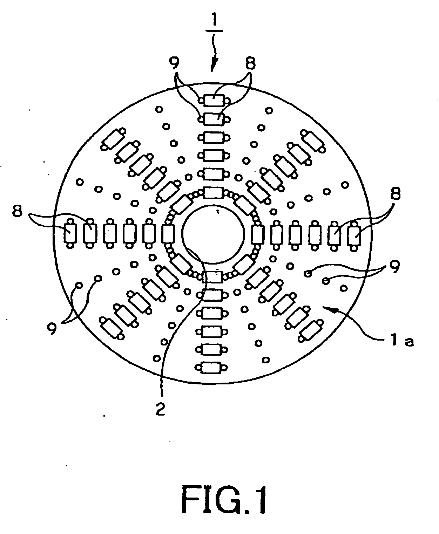

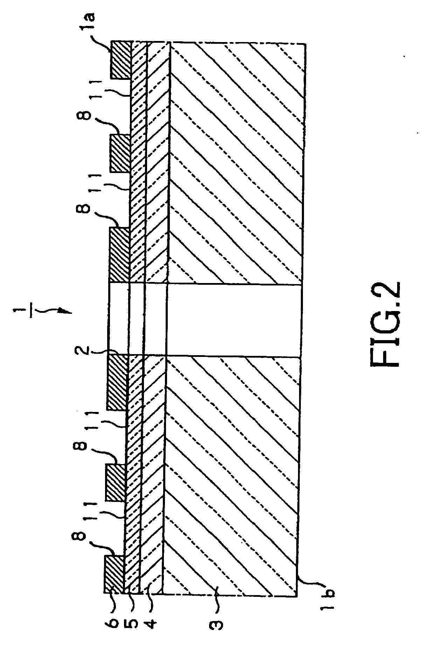

Referring now to FIG. 1, there is schematically illustrated the top of a bioassay substrate 1 as an embodiment of the present invention. FIG. 2 is a schematic sectional view of the bioassay substrate 1 in FIG. 1.

The bioassay substrate 1 is flat and generally formed to have a disc-shaped main side like an optical disc such as CD (Compact Disc), DVD (Digital Versatile Disc) or the like. The bioassay substrate 1 has formed in the center thereof a central hole 2 in which a chucking mechanism for holding and rotating the bioassay substrate 1 is to be inserted when the bioassay substrate 1 is loaded in a DNA analyzer.

As shown in FIG. 2, the bioassay substrate 1 includes, counting from below, a base layer 3, transparent electrode layer 4, solid-phasing layer 5 and a well-forming layer 6. It should be noted that the surface of the bioassay substrate 1 at the well-forming layer 6 will be referred to as "upper surface 1a" and the surface at the base layer 3 be referred to as "lower surface 1b" hereinbelow.

The base layer 3 is transparent for light that excites a fluorescent marker that will be described in detail later and fluorescence of the fluorescent marker. For example, the base layer 3 is formed from a material such as quartz glass, silicon, polycarbonate, polystyrene or the like.

The transparent electrode layer 4 is formed on the base layer 3. The transparent electrode layer 4 is formed from a light-transparent, electroconductive material such as ITO (indium-tin-oxide), aluminum or the like, for example. The transparent electrode layer 4 is a film formed on the base layer 3 by, for example, sputtering or electron beam evaporation to a thickness of about 250 nm.

The solid-phasing layer 5 is formed on the transparent electrode layer 4. The solid-phasing layer 5 is formed from a material that solid-phases one end of the probe DNA. In this embodiment, the solid-phasing layer 5 is a film made of SiO₂ by, for example, sputtering or electron beam evaporation, to a thickness of about 50 nm. The surface of the solid-phasing layer 5 can be modified at the surface thereof with silane.

The well-forming layer 6 is formed on the solid-phasing layer 5. It has a plurality of wells 8 formed therein.

The inner space of each well is a place in which a probe DNA (detection-use nucleotide chain) and sample DNA (target nucleotide chain) react with each other, more specifically, a hybridization field. The well 8 is a concavity open at the upper surface 1a of the bioassay substrate 1 and having a sufficient depth and size to hold a liquid dripped into the well 8, such as a solution containing the sample DNA. For example, the well 8 has an opening of 100 µm in length of each side, a depth of about 5 µm and a bottom 11 through which the solid-phasing layer 5 is exposed.

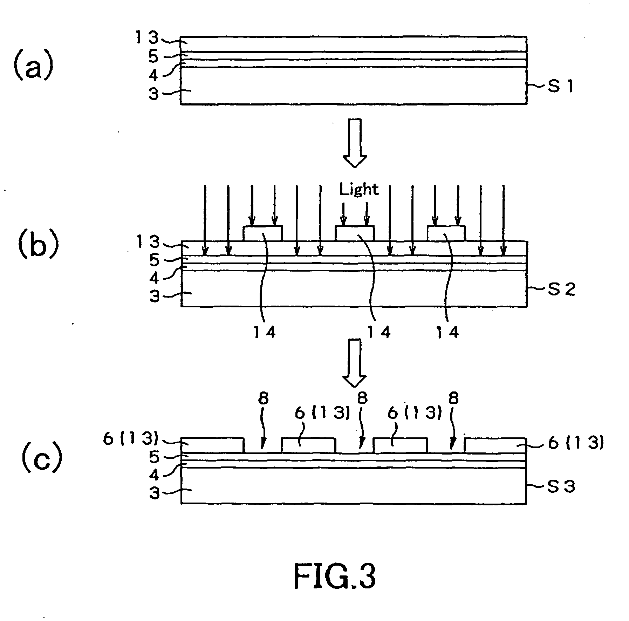

The well-forming layer 6 is formed as will be described below. First, photosensitive polyimide 13 is applied to over the solid-phasing layer 5 by spin coating or the like to a thickness of about 5 µm (in step S 1) as shown in FIG. 3(a). Next, a photomask 14 of a predetermined pattern is formed on the photosensitive polyimide 13 applied as above and the photosensitive polyimide 13 with the photomask 14 is exposed to light and developed (in step S2), as shown in FIG. 3(b). Thus, the plurality of wells 8 is formed on the well-forming layer 6 (in step S3) as shown in FIG. 3(c).

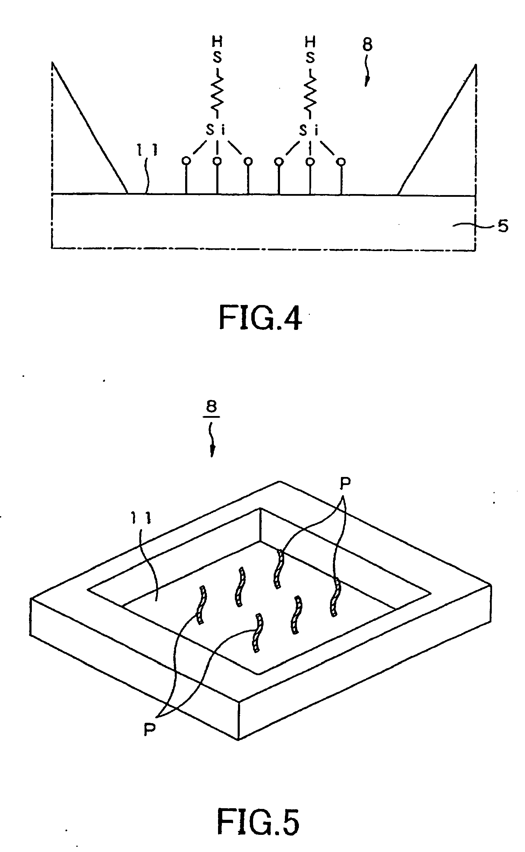

Further, the well 8 is surface-modified at the bottom 11 thereof with a functional group so that the probe DNA modified at one end thereof with a functional group will combine with the bottom 11 (in which the solid-phasing layer 5 is exposed). For example, the well 8 is surface-modified at the bottom 11 thereof (solid-phasing layer 5 made of SiO₂) with silane molecules 16 each having an SH group 15 as shown in FIG. 4. Thus, the probe DNA modified at one end thereof with, for example, an SH group, can be combined with the bottom 11 of the well 8. As above, in the bioassay substrate 1, since the probe DNA can be combined at one end thereof with the bottom 11 of the well 8, the probe DNA (P) can be combined so that its chain extends vertically from the bottom 11 as shown in FIG. 5.

Also, in the bioassay substrate 1, a plurality of wells 8 is disposed at regular intervals of about 400 µm, for example, on a plurality of radially extending arrays from the center of the main side toward the outer radius as shown in FIG. 1.

Also, the bioassay substrate 1 has formed thereon address pits 9 that can be read by irradiating laser light from the lower surface 1b of the bioassay substrate 1. The address pits 9 are information intended for locating the wells 8 in the plane of the bioassay substrate 1. By optically reading information from the address pits 9, it is possible to locate which one of the plurality of wells 8 that is currently being irradiated with the laser light. Because of the address pits 9 thus formed on the bioassay substrate 1, it is possible to control the position of solution dripping by a dripping apparatus which will be described in detail later and locate the fluorescence detected by an objective lens.

Since the aforementioned bioassay substrate 1 is disc-shaped, a playback system similarly to an optical disc system can be used to make focusing servo control for controlling focused position of laser light, positioning servo control for controlling irradiated position of laser light in relation to the radial direction and position of dripping from the dripping apparatus, and detect information from the address pits 9. More specifically, with information recorded at the address pits 9 having been pre-associated with the wells 8 near the address pits 9, a specific one of the wells 8 irradiated with laser light and emitting fluorescence can be located by reading information from the address pit 9 corresponding to the specific well 8 and a solution can be dripped into the well 8 by reading information from the address pit 9 corresponding to the well 8 and controlling the relative position between the well 8 and the dripping apparatus.

In addition, the above-mentioned bioassay substrate 1 can have a parallel electric field formed between an electrode and transparent electrode layer 4 by placing the electrode in a position near the transparent electrode layer 4 from above the well 8. Thus, in hybridizing DNAs, it is possible to promote the hybridization of the DNAs in the well 8 by applying an AC electric field to the well 8 to elongate the DNAs drifting in the well 8.

Next, there will be explained DNA analysis using the aforementioned bioassay substrate 1.

First, a solution S containing a probe DNA modified at one end thereof with an SH group is dripped into a predetermined well 8. At this time, a plurality of types of probe DNA will be dripped onto one bioassay substrate 1. However, one type of probe DNA has to be dripped into one well 8. It should be noted that this dripping of one type of probe DNA into one well 8 is controlled based on a location map prepared in advance and indicating a correspondence between a well and probe DNA.

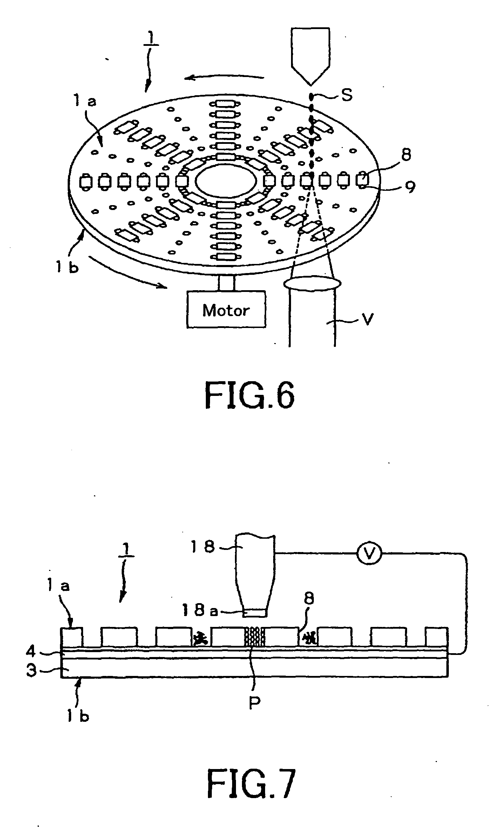

Also, dripping of the solution S is controlled by moving the bioassay substrate 1 as in the optical disc driving system. More specifically, the dripping position should be controlled by locating a well 8 to which the solution S is to be dripped and a corresponding address pit 9 through rotation of the bioassay substrate 1 while being held in parallel with the upper surface 1a upside and irradiation of laser light V from below (from the lower surface 1b) the bioassay substrate 1, as shown in FIG. 6.

Next, a probe-shaped external electrode 18 having formed at the free end thereof a flat surface 18a sufficiently larger than the opening of a predetermined well 8 (a disc-like surface of 300 µm in diameter, for example) is moved from outside and toward the upper surface 1a of the bioassay substrate 1 until the free end will cover the well 8, as shown in FIG. 7. Then, an AC voltage is applied to between the external electrode 18 and transparent electrode layer 4 to apply an AC electric field perpendicular to the main side of the bioassay substrate 1. For example, an AC electric field of about 1 MV/m and 1 MHz is applied to inside the well 8.

With the AC electric field being applied to the predetermined well 8 as above, the probe DNA (P) drifting in the solution in the well 8 is elongated perpendicularly to the main side of the bioassay substrate 1 and the probe DNA moves perpendicularly to the bioassay substrate 1. Thus, the probe DNA can be solid-phased (fixed) to the already surface-modified bottom 11 of the well 8 with the modified end of the probe DNA being combined with the bottom 11.

To apply a parallel electric field perpendicularly to the main side of the bioassay substrate 1, the flat surface 18a should desirably be formed at the free end of the external electrode 18 and parallel to the transparent electrode layer 4. Also, to assure the flatness of the surface 18a, a mirror-finished semiconductor wafer of Si or GaAs having acceptor or donor ions doped at a high concentration therein may be installed to a probe-shaped metal free end of the external electrode 18. In installing the semiconductor wafer, the Schottky barrier between the probe-shaped metal and semiconductor wafer should desirably be formed smaller and the semiconductor wafer be connected with a titanium or gold laid between itself and the probe-shaped metal free end of the external electrode 18 for an ohmic contact.

The application of the AC electric field leads to elongation and movement of the single-strand DNA (nucleotide chain) for the following reason. That is, it is inferred that in the nucleotide chain, ion cloud is formed from phosphoric ions (negative charge) as the core of the nucleotide chain, and hydrogen ions (positive charge) resulted from ionization of water surrounding the phosphoric ions. The negative and positive charges result in a vector of polarization. The polarization vector as a whole will be oriented in one direction due to the application of a high-frequency, high voltage, with the result that the nucleotide chain will be elongated. Further, if a nonuniform electric field in par of which electric flux lines are concentrated is applied, the nucleotide chain will also move toward the part of the electric field to which the electric flux lines are concentrated (cf. Seiichi Suzuki, Takeshi Yamanashi, Shin-ichi Tazawa, Osamu, Kurosawa and Masao Washizu - Quantitative Analysis on Electrostatic Orientation of DNA in Stationary AC electric field Using Fluorescence Anisotropy & Quot. -IEEE Transaction on Industrial Application, Vol. 34, No. 1, pp. 75 - 83 (1998)).

As above, when an AC electric field is applied, the probe DNA will be elongated in a direction parallel to the electric field, resulting in a state of less steric hindrance, in which the probe DNA and bottom 11 are easily combined with each other. Then, with this combination between the probe DNA and bottom 11, the probe DNA can be solid-phased (fixed) to the bottom 11 of the well 8.

Next, a solution containing a sample DNA extracted from a living organism is dripped into each of the wells 8 of the bioassay substrate 1.

Then, after dripping of the sample DNA, the external electrode 18 is moved from outside the upper surface 1a of the bioassay substrate 1 to cover a predetermined one of the wells 8. Next, an AC voltage is applied to between the external electrode 18 and transparent electrode layer 4 with the temperature being kept at about 60°C. That is, while the bioassay substrate 1 is being heated, the AC electric field is applied perpendicularly to the main side of the bioassay substrate 1. The AC electric field to be applied to inside the well 8 is, for example, of about 1 MV/m and 1 MHz.

With the above operations, the sample and probe DNAs are elongated perpendicularly to have a state of less steric hindrance, and the sample DNA moves in a direction perpendicular to the bioassay substrate 1. As a result, in case a sample DNA and probe DNA having a complementary relation in base sequence between them coexist in the same well 8, they will be hybridized.

Then, after the hybridization, a fluorescence marking intercurator or the like is dripped into the well 8 of the bioassay substrate 1. Such a fluorescence marking intercurator is inserted into a double helix between the hybridized probe and sample DNAs to combine the DNAs with each other.

Next, the surface 1a of the bioassay substrate 1 is washed with deionized water or the like to remove the sample DNA and fluorescent marker from inside the well 8 in which no hybridization has occurred. As a result, the fluorescent marker will remain in only the well 8 where the hybridization has occurred.

Then, fluorescence from the well 8 is detected by controlling the movement of the bioassay substrate 1 as in the optical disc driving system. More specifically, the well 8 is located by rotating the bioassay substrate 1 while holding it and irradiating laser light V from below (from the lower surface 1b) of the bioassay substrate 1 to detect a corresponding address pint 9. At the same time, excitation light is irradiated from below (from the lower surface 1b) of the bioassay substrate 1 and fluorescence developed at the lower surface 1b correspondingly to the irradiated excitation light is detected. It is thus detected from which well 8 the fluorescence comes.

Next, there is prepared a map indicating the position of the well 8 on the bioassay substrate 1 from which the detected fluorescence has come. Then, the base sequence of the sample DNA is analyzed based on the prepared map and a location map indicating the type of the base sequence of the probe DNA dripped into each well 8.

In the above DNA analysis using the bioassay substrate 1, one end of the probe DNA drifting in the solution is connected to the bottom 11 of the well 8 while the probe DNA is elongated and moved perpendicularly by applying an AC electric field perpendicular to the surface of the bioassay substrate 1. Since the electric field is applied perpendicularly to the bioassay substrate 1, the electrode may not be generated by patterning it on the substrate, for example, so that an electrode having an extremely simple layer structure can be used to fix the probe DNA.

Also, in the DNA analysis using the aforementioned bioassay substrate 1, the sample DNA drifting in the solution and probe DNA fixed at one end thereof to the bottom of the well 8 are elongated and moved perpendicularly by applying a perpendicular AC electric field to the DNAs. Therefore, since the sample and probe DNAs are elongated and moved both in the same direction, the electrode for applying such an electric field may not be formed by patterning it on the substrate, so that the probe DNA can be fixed using an electrode of which the layer structure is very simple.

Note that although in the embodiment of the present invention, the external electrode 18 is shaped like a probe and the AC electric field is applied to only a smaller number of wells 8, the external electrode 18 is not limited to the one having the probe shape but may have any shape which would be capable of applying a perpendicular AC electric field to the well 8. For example, a disc-shaped electrode almost equal in size to the main side of the bioassay substrate 1 may be used to apply an AC electric field to all the wells 8 at the same time. Also, although the transparent electrode layer 4 is provided in the bioassay substrate 1 in this embodiment, the transparent electrode layer 4 may not be provided so and an electric field may be applied perpendicularly to the well 8 by moving a similar electrode to the external electrode 18 to the bioassay substrate 1 from outside the lower surface 1b.

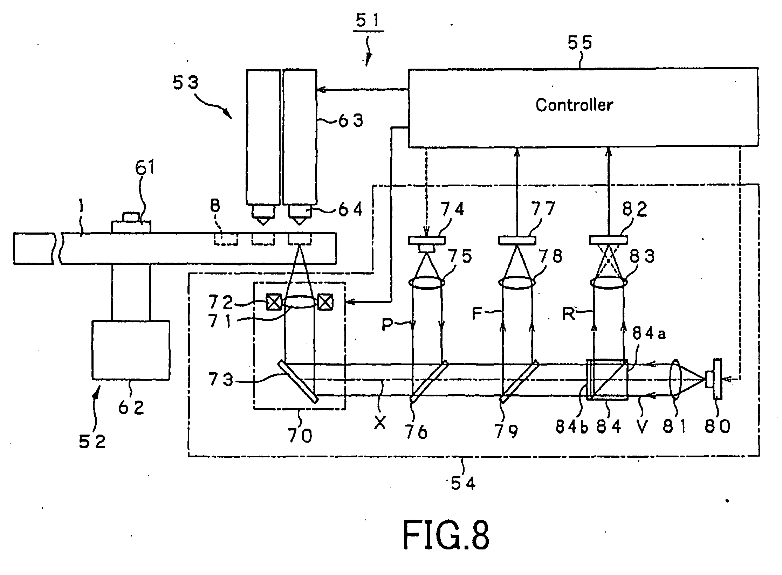

Next, a DNA analyzer 51 to make DNA analysis with the use of the bioassay substrate 1 according to the present invention will be described below with reference to FIG. 8.

As shown in FIG. 8, the DNA analyzer 51 includes the external electrode 18, a disc loader 52 to hold and rotate the bioassay substrate 1, a dripping unit 53 to store a variety of solutions for use in hybridization and drip the solution into the well 8 of the bioassay substrate 1, an excitation light detector 54 to detect excitation light from the bioassay substrate 1, and a controller 55 to manage and control the above components.

The disc loader 52 includes a chucking mechanism 61 to be inserted into the central hole 2 in the bioassay substrate 1 and hold the bioassay substrate 1, and a spindle motor 62 to rotate the bioassay substrate 1 by driving the chucking mechanism 61. The disc loader 52 rotates the bioassay substrate 1 while holding the bioassay substrate 1 horizontally with the upper surface 1a upside. The disc loader 52 does not incur any drip-off of the solution dripped into the well 8 by holding the bioassay substrate 1 horizontally.

The dripping unit 53 includes a reservoir 63 to store sample solution S and fluorescent marker S', and a dripping head 64 to drip the sample solution S and fluorescent marker S' from the reservoir 63 onto the bioassay substrate 1. The dripping head 64 is disposed above the upper surface 1a of the bioassay substrate 1 loaded horizontally. Further, the dripping head 64 is designed to control the position relative to the bioassay substrate 1 radially on the basis of positional information and rotation synchronization information read from the address pits on the bioassay substrate 1 to accurately track a reaction area of a predetermined well 8 and drip the sample solution S containing a sample DNA (target nucleotide chain T) onto the reaction area. Also, the reservoir 63 and dripping head 64 can be combined with each other in as many ways as sample solutions used in hybridization.

Also, the dripping unit 53 adopts the so-called "ink-jet printing" technique, for example, to accurately drip the sample solution S to a predetermined position on the bioassay substrate 1. With the "ink-jet printing" technique, an ink jet mechanism used in the so-called ink-jet printer is adopted in the dripping unit 64, and the sample solution S is sprayed from a nozzle head as in the ink-jet printer to the bioassay substrate 1.

The excitation light detector 54 has an optical head 70. The optical head 70 is disposed below the bioassay substrate 1 loaded horizontally, namely, at the lower surface 1b. The optical head 70 can freely be moved by a sled mechanism (not shown), for example, radially of the bioassay substrate 1.

The optical head 70 includes an objective lens 71, biaxial actuator 72 supporting the objective lens 71 to be movable, and a light guiding mirror 73. The objective lens 71 is supported on the biaxial actuator 72 for its central axis to be almost perpendicular to the surface of the bioassay substrate 1. Therefore, the objective lens 71 can focus a light beam incident from below the bioassay substrate 1 on the latter. The biaxial actuator 72 supports the objective lens 71 to be movable in two directions, that is, perpendicularly to the surface of the bioassay substrate 1 and radially of the bioassay substrate 1. By driving the biaxial actuator 72, the spot defined by the light focused by the objective lens 71 can be moved perpendicularly to the surface of the bioassay substrate 1 and radially of the latter. Therefore, the optical head 70 can be controlled in the similar manner to the just-focus control and positioning control as in the optical disc system.

The light guiding mirror 73 is disposed at an angle of 45 deg. in relation to an optical path X along which the excitation light P, fluorescence F, servo light V and return light R are incident upon the optical head 70 and go out of the latter. The excitation light P and servo light V are incident upon the light guiding mirror 73 from the optical path X. The light guiding mirror 73 refracts, by reflection, the excitation light P and servo light V through an angle of 90 deg. for incidence upon the objective lens 71. The excitation light P and servo light V incident upon the objective lens 71 are condensed by the latter for irradiation to the bioassay substrate 1. Also, from the bioassay substrate 1, the fluorescence F and reflected component (return light) R of the servo light V are incident upon the light guiding mirror 73 through the objective lens 71. The light guiding mirror 73 refracts, by reflection, the fluorescence F and return light R through an angle of 90 deg. for going along the optical path X.

Note that a drive signal to sled the optical head 70 and a drive signal to drive the biaxial actuator 72 are supplied from the controller 55.

Also, the excitation light detector 54 includes an excitation light source 74 to emit excitation light P, collimator lens 75 to form the excitation light P emitted from the excitation light source 74 into a parallel light beam, and a first dichroic mirror 76 to refract the excitation light P formed into the parallel light beam by the collimator lens 75 on the optical path X for irradiation to the light guiding mirror 73.

The excitation light source 74 is to emit laser light having such a wavelength that can excite the fluorescent marker. In the present invention, the excitation light P emitted from the excitation light source 74 is laser light whose wavelength is 405 nm. It should be noted that the wavelength of the excitation light P may be any one that would be able to excite the fluorescent marker. The collimator lens 75 forms the excitation light P emitted from the excitation light source 74 into a parallel light beam. The first dichroic mirror 76 is a wavelength-selective reflecting mirror that will reflect only light whose wavelength is equal to that of the excitation light P while allows light whose wavelength is equal to that of the fluorescence F and servo light V (its return light R) to pass by. The first dichroic mirror 76 is inserted in the optical path X at an angle of 45 deg. to refract, by reflection, the excitation light P coming from the collimator lens 75 through an angle of 90 deg. for irradiation to the light guiding mirror 73.

Also, the excitation light detector 54 includes an avalanche photodiode 77 to detect the fluorescent F, condenser lens 78 to condense the fluorescence F, and a second dichroic mirror 79 to refract the fluorescence F coming to the optical path X from the optical head 70 for irradiation to the avalanche photodiode 77.

The avalanche photodiode 77 is highly sensitive to detect the fluorescence F whose intensity is low. It should be noted that the avalanche photodiode 77 can detect the fluorescence F having a wavelength of about 470 nm. Also, the wavelength of the fluorescence F varies depending upon the type of a fluorescent marker used. The condenser lens 78 is to condense the fluorescence F onto the avalanche photodiode 77. The second dichroic mirror 79 is inserted in the optical path X at an angle of 45 deg. and disposed downstream of the first dichroic mirror 76 when viewed from the light guiding mirror 73. Therefore, the fluorescence F, servo light V and return light R will be incident upon the second dichroic mirror 79, but the excitation light P will not. The second dichroic mirror 79 is a wavelength-selective reflecting mirror to reflect only light whose wavelength is equal to that of the fluorescence F while allowing light whose wavelength equal to that of the servo light V (return light R). The second dichroic mirror 79 refracts, by reflection, the fluorescence F coming from the light guiding mirror 73 of the optical head 70 through an angle of 90 deg. for irradiation to the avalanche photodiode 77 through the condenser lens 78.

The avalanche photodiode 77 generates an electric signal corresponding to the intensity of the fluorescence F thus detected, and supplies it to the controller 55.

The excitation light detector 54 includes a servo light source 80 to emit servo light V, collimator lens 81 to form the servo light V emitted from the servo light source 80 into a parallel light beam, photodetector circuit 82 to detect return component R of the servo light V, cylindrical lens 83 to cause astigmatism in order to condense the return light R to the photodetector circuit 82, and a light separator 84 to separate the servo light V and return light R from each other.

The servo light source 80 has a laser source to emit laser light whose wavelength is, for example, 780 nm. It should be noted that the servo light V has a wavelength with which the address pint can be detected. The wavelength is not limited to 780 nm but may be any one that is different from those of the excitation light P and fluorescence F. The collimator lens 81 forms the servo light V emitted from the servo light source 80 into a parallel light beam. The servo light V thus formed into the parallel light beam is incident upon the light separator 84.

The photodetector circuit 82 includes a detector to detect the return light R, and a signal generation circuit to generate a focus error signal, positioning error signal and address pit read signal from the detected return light R. Since the return light R is a component of the servo light V reflected by the bioassay substrate 1, its wavelength is 780 nm that is equal to that of the servo light V.

Note that the focus error signal indicates a displacement between the position of the light focused by the objective lens 71 and the base layer 3 of the bioassay substrate 1. When the focus error signal is zero (0), it is meant that the distance between the objective lens 71 and bioassay substrate 1 is optimum. The positioning error signal indicates a disc-radial displacement between the position of a predetermined well 8 and light-focused position. When the positioning error signal is zero (0), it is meant that the disc-radial irradiated position of the servo light V coincides with an arbitrary one of the wells 8. The address pit read signal indicates information recorded at the address pits formed on the bioassay substrate 1. By reading the information, it is possible to locate a well 8 currently being irradiated with the servo light V.

The photodetector circuit 82 supplies the controller 55 with the focus error signal, positioning error signal and address pit read signal all base on the return light R.

The cylindrical lens 83 is to focus the return light R on the photodetector circuit 82 and cause an astigmatism. By causing such an astigmatism, the photodetector circuit 82 can generate a focus error signal.

The light separator 84 includes a light separating surface 48a formed from a polarizing beam splitter and a quarter waveplate 84b. Light incident upon a side of the light separator 84 opposite to the quarter waveplate 84b will be transmitted through the light separating surface 84a, and return component of the transmitted light, incident upon the quarter waveplate 84b, will be reflected by the light separating surface 84a. The light separator 84 has the light separating surface 84a thereof inserted in the optical path X at an angle of 45 deg. and disposed downstream of the second dichroic mirror 79 when viewed from the light guiding mirror 73. Therefore, the light separator 84 allows the servo light V coming from the collimator lens 81 to pass by and be incident upon the light guiding mirror 73 in the optical head 70, while refracting, by reflection, the return light R coming from the light guiding mirror 73 in the optical head 70 through an angle of 90 deg. for irradiation to the photodetector circuit 82 through the cylindrical lens 83.

The controller 55 makes a variety of servo control operations on the basis of the focus error signal positioning error signal and address pit read signal detected by the excitation light detector 54.

More specifically, the controller 55 provides servo control to zero the focus error signal by driving the biaxial actuator 72 in the optical head 70 on the basis of the focus error signal to control the interval between the objective lens 71 and bioassay substrate 1. Also, the controller 55 provides servo control to zero the focus error signal by driving the biaxial actuator 72 in the optical head 70 on the basis of the positioning error signal to move the objective lens 71 radially of the bioassay substrate 1. In addition, the controller 55 sleds the optical head 70 on the basis of the address pit read signal to move the optical head 70 to a predetermined radial position, thereby moving the objective lens 71 to the position of a target well.

Also, at the time of hybridization, the controller 55 controls an AC power generator 31 to control the power supply as well.

The DNA analyzer 51 constructed as above operates as will be described below:

In the DNA analyzer 51, a solution containing a sample DNA is dripped into the well 8 with the bioassay substrate 1 being rotated, to have a probe DNA in the well 8 and the sample DNA react with each other (hybridization). During the hybridization, the aforementioned electric field is also controlled. Also, a buffer solution containing a fluorescent marker onto the bioassay substrate 1 where the hybridization has been completed.

Also, in the DNA analyzer 51, the bioassay substrate 1 having the fluorescent marker dripped thereon is rotated, the excitation light P is incident from the lower surface 1b of the bioassay substrate 1 for irradiation to the fluorescent marker in the well 8, and the fluorescence F taking place from the fluorescent marker correspondingly to the excitation light P is detected from below the bioassay substrate 1.

In the DNA analyzer 51, the excitation light P and servo light V are irradiated to the bioassay substrate 1 through the same objective lens 71. Thus, the DNA analyzer 51 can identify the irradiated position of the excitation light P, that is, the emitting position of the fluorescence F by controlling the focus, positioning and address with the use of the servo light V, and identify a probe DNA combined with the sample DNA on the basis of the position from which the fluorescence is emitted.

In the foregoing, the present invention has been described in detail concerning certain preferred embodiments thereof as examples with reference to the accompanying drawings. However, it should be understood by those ordinarily skilled in the art that the present invention is not limited to the embodiments but can be modified in various manners, constructed alternatively or embodied in various other forms.

### Industrial Applicability

In the biochemical reaction apparatus according to the present invention, an electrode is moved toward a substrate having a reaction area where biochemical reaction takes place and an electrode formed in the reaction area to form a parallel electric field in the reaction area. Thus, in hybridization of a nucleotide chain, for example, an AC electric field is applied to a well to elongate the nucleotide chain drifting in the well and thus promote the hybridization.

The biochemical reaction substrate according to the present invention includes an electrode to generate an electric field between itself and an external electrode to form an electric field in a reaction area. Therefore, in this biochemical reaction substrate, a parallel electric field can be formed in the reaction field by moving the electrode toward the reaction area. Thus, in hybridization of a nucleotide chain, for example, an AC electric field is applied to the well to elongate the nucleotide chain drifting in the well and thus promote the hybridization.

In the substrate producing method according to the present invention, an AC electric field is applied perpendicularly to the surface of the substrate to elongate and move a probe-use nucleotide chain perpendicularly in order to connect one end of the nucleotide chain to the flat substrate surface. Therefore, in this substrate producing method, since the electric field is applied perpendicularly to the flat substrate, the probe-use nucleotide chain can be fixed to the substrate at a high speed using an electrode having a very simple construction.

In the hybridizing method according to the present invention, a probe-use nucleotide chain is fixed in a well with one end thereof being connected to the surface of the flat substrate, an AC electric field is applied perpendicularly to the flat substrate surface to elongate and move the nucleotide chain in the well perpendicularly. Therefore, in this hybridizing method, since the electric field is applied perpendicularly to the flat substrate, the probe-use nucleotide chain can be fixed to the substrate at a high speed using an electrode having a very simple construction.

## Claims

1. A biochemical reaction apparatus (51) using a biochemical reaction substrate, the apparatus comprising:
a means (52) for holding a substrate (1) having a reaction area (8) for biochemical reaction and an electrode (4) formed in the reaction area;
an external electrode (18) disposed opposite to the electrode (4) of the substrate; and
an electric field controlling means (55) for generating an electric field between the electrode (4) of the substrate and external electrode (18) wherein :
the electrode (4) of the substrate is a conductive layer included in the substrate and formed as an underlying layer of the reaction area; and the external electrode (18) has a plane parallel to the conductive layer.

2. The apparatus according to claim 1, wherein the electric field controlling means (55) generates an AC electric field between the substrate electrode (4) and external electrode (18).

3. The apparatus according to claim 1, wherein the external electrode (18) is formed like a probe.

4. The apparatus according to claim 1, wherein the external electrode (18) is formed from a semiconductor having acceptor or donor ions doped therein.

5. A biochemical reaction substrate (1) used for biochemical reaction, the substrate comprising:
a reaction area (8) for biochemical reaction; and
an electrode (4) for generating an electric field between itself and an external electrode (18) for the electric field to be formed inside the reaction area; wherein:
the biochemical reaction is a hybridization reaction of a nucleotide chain;
the reaction area has a surface coat (5) internally processed for the nucleotide chain to be fixable thereon; and
the electrode (4) is a conductive layer included in the substrate and formed as an underlying layer of the surface coat.

6. The biochemical reaction substrate according to claim 5, wherein the conductive layer is formed in the reaction area as an underlying layer of the reaction area so that the electric field generated between itself (4) and external electrode (18) is formed almost perpendicularly to the surface coat.

7. The biochemical reaction substrate according to claim 5, wherein the conductive layer forms an electric field between itself and an electrode disposed in a position opposite to the surface coat.

8. The biochemical reaction substrate according to claim 5, wherein the substrate is disc-shaped and has reading control information recorded therein.

9. The biochemical reaction substrate according to claim 5, wherein the conductive layer is light-transparent.

10. A method of producing a hybridization substrate (1), the method comprising the steps of:
forming, on the flat surface of a substrate according to claim 1, a plurality of wells (8) each modified at the bottom thereof with a first functional group;
dripping, into each well, a solution containing a nucleotide chain modified at one end thereof with a second functional group that combines with the first functional group; and
combining the first function group with the second functional group while applying an AC electric field perpendicular to the flat substrate to combine the nucleotide chain with the bottom of the well; wherein:
the flat substrate has formed as an underlying layer of the well an electrode layer (4) formed from an electrically conductive material; and
an external electrode (18) is provided near the substrate surface to apply an AC power to between the external electrode and electrode layer in order to apply an AC electric field perpendicularly to the flat substrate.

11. The method according to claim 10, wherein the external electrode is formed from a semiconductor having acceptor or donor ions doped therein.

12. A hybridizing method comprising the steps of:
dripping a solution containing a sample-use nucleotide chain into a well (8) formed on the surface of a flat substrate according to claim 1 and having one end of a probe-use nucleotide chain combined with the bottom thereof; and
hybridizing the probe-use nucleotide chain and sample-use nucleotide chain while applying an AC electric field perpendicularly to the flat substrate; wherein:
the flat substrate has formed as an underlying layer of the well an electrode layer formed from an electrically conductive material; and
an external electrode is provided near the substrate surface to apply an AC power to between the external electrode and electrode layer in order to apply an AC electric field perpendicularly to the flat substrate.

13. The method according to claim 12, wherein the external electrode is formed from a semiconductor having acceptor or donor ions doped therein.

## Patentansprüche

1. Biochemische Reaktionsvorrichtung (51), bei der ein biochemisches Reaktionssubstrat verwendet wird, wobei die Vorrichtung umfasst:
eine Einrichtung (52) zum Halten eines Substrats (1), welches einen Reaktionsbereich (8) für biochemische Reaktion hat, und einer Elektrode (4), welche im Reaktionsbereich gebildet ist;
eine externe Elektrode (18), welche gegenüber der Elektrode (4) des Substrats angeordnet ist; und
eine elektrische Feldsteuereinrichtung (55) zum Erzeugen eines elektrischen Felds zwischen der Elektrode (4) des Substrats und der externen Elektrode (18), wobei:
die Elektrode (4) des Substrats eine leitfähige Schicht ist, welche im Substrat vorhanden ist und als eine darunter liegende Schicht des Reaktionsbereichs gebildet ist; und die externe Elektrode (18) eine Ebene parallel zur leitfähigen Schicht hat.

2. Vorrichtung nach Anspruch 1, wobei die elektrische Feldsteuereinrichtung (55) ein elektrisches Wechselfeld zwischen der Substratelektrode (4) und der externen Elektrode (18) erzeugt.

3. Vorrichtung nach Anspruch 1, wobei die externe Elektrode (18) als eine Sonde ausgebildet ist.

4. Vorrichtung nach Anspruch 1, wobei die externe Elektrode (18) von einem Halbleiter gebildet ist, der Akzeptor- oder Donator-Ionen hat, die darin dotiert sind.

5. Biochemisches Reaktionssubstrat (1), welches für biochemische Reaktion verwendet wird, wobei das Substrat umfasst:
einen Reaktionsbereich (8) für biochemische Reaktion; und
eine Elektrode (4) zum Erzeugen eines elektrischen Felds zwischen ihr und einer Elektrode (18) für das elektrische Feld, welches innerhalb des Reaktionsbereichs zu bilden ist; wobei:
die biochemische Reaktion eine Hybridisierung-Reaktion einer Nukleotid-Kette ist;
der Reaktionsbereich einen Flächenüberzug (5) hat, der intern für die Nukleotid-Kette bearbeitet ist, um darauf flexibel zu sein; und
die Elektrode (4) eine leitfähige Schicht ist, welche im Substrat vorhanden ist und als eine darunter liegende Schicht des Flächenüberzug ausgebildet ist.

6. Biochemisches Reaktionssubstrat nach Anspruch 5, wobei die leitfähige Schicht im Reaktionsbereich als eine darunter liegende Schicht des Reaktionsbereichs gebildet ist, so dass das elektrische Feld, welches zwischen ihr (4) und der externen Elektrode (18) erzeugt wird, beinahe senkrecht zum Flächenüberzug ausgebildet ist.

7. Biochemisches Reaktionssubstrat nach Anspruch 5, wobei die leitfähige Schicht ein elektrisches Feld zwischen ihr und einer Elektrode, welche in einer Position gegenüber dem Flächenüberzug angeordnet ist, bildet.

8. Biochemisches Reaktionssubstrat nach Anspruch 5, wobei das Substrat plattenförmig ist und Lesesteuerinformation, welche darin aufgezeichnet ist, hat.

9. Biochemisches Reaktionssubstrat nach Anspruch 5, wobei die leitfähige Schicht licht-transparent ist.

10. Verfahren zum Erzeugen eines Hybridisierungs-Substrats (1), wobei das Verfahren folgende Schritte umfasst:
Bilden - auf der flachen Fläche des Substrats - nach Anspruch 1 mehrerer Schächte (8), wobei jede an ihrem Boden mit einer ersten Funktionsgruppe modifiziert ist;
Tropfen lassen - in jeden Schacht - einer Lösung, welche eine Nukleotid-Kette enthält, welche an ihrem einen Ende mit einer zweiten Funktionsgruppe modifiziert ist, welche sich mit der ersten Funlctionsgruppe verbindet; und
Verbinden der ersten Funktionsgruppe mit der zweiten Funktionsgruppe, während ein elektrisches Wechselfeld senkrecht zur flachen Fläche angelegt wird, um die Nukleotid-Kette mit dem Boden des Schachts zu verbinden; wobei:
die flache Fläche - als eine darunterliegende Schicht des Schachts - eine Elektrodenschicht (4) hat, welche von einem elektrischen leitfähigen Material gebildet ist, und
eine externe Elektrode (18) in der Nähe der Substratfläche vorgesehen ist, um eine Wechselspannung zwischen der externen Elektrode und der Elektrodenschicht anzulegen, um ein elektrisches Wechselfeld senkrecht zur flachen Fläche anzulegen.

11. Verfahren nach Anspruch 10, wobei die externe Elektrode aus einem Halbleiter gebildet ist, der Akzeptor- und Donator-Ionen, die darin dotiert sind, hat.

12. Hybridisierungsverfahren, welches folgende Schritte umfasst:
Tropfen lassen einer Lösung, welche eine Probenverwendungs-Nukleotid-Kette enthält, in einen Schacht (8), welcher auf der Fläche des flachen Substrats gebildet ist, nach Anspruch 1, und welcher ein Ende einer Sondenverwendungs-Nukleotid-Kette hat, welche mit ihrem Boden verbunden ist; und
Hybridisieren der Sondenverwendungs-Nulcleotid-Kette und der Probenverwendungs-Nulcleotid-Kette, während ein elektrisches Wechselfeld senkrecht zur flachen Fläche angelegt wird; wobei:
das flache Substrat, welches als eine darunterliegende Schicht des Schachts gebildet ist, eine Elektrodenschicht hat, welche von einem elektrischen leitfähigen Material gebildet ist; und
eine externe Elektrode in der Nähe der Substratfläche vorgesehen ist, um eine Wechselspannung zwischen der externen Elektrode und der Elektrodenschicht anzulegen, um ein elektrisches Wechselfeld senkrecht zur flachen Fläche anzulegen.

13. Verfahren nach Anspruch 12, wobei die externe Elektrode von einem Halbleiter gebildet wird, der Akzeptor- und Donator-Ionen, die darin dotiert sind, hat.

## Revendications

1. Appareil de réaction biochimique (51) utilisant un substrat de réaction biochimique, l'appareil comprenant :
un moyen (52) pour maintenir un substrat (1) ayant une zone de réaction (8) pour une réaction biochimique et une électrode (4) formée dans la zone de réaction ;
une électrode externe (18) disposée de manière opposée à l'électrode (4) du substrat ; et
un moyen de commande de champ électrique (55) pour générer un champ électrique entre l'électrode (4) du substrat et l'électrode externe (18)
où :
l'électrode (4) du substrat est une couche conductrice incluse dans le substrat et formée comme une couche sous-jacente de la zone de réaction ; et l'électrode externe (18) a un plan parallèle à la couche conductrice.

2. Appareil selon la revendication 1, où le moyen de commande de champ électrique (55) génère un champ électrique CA entre l'électrode du substrat (4) et l'électrode externe (18).

3. Appareil selon la revendication 1, où l'électrode externe (18) est formée comme une sonde.

4. Appareil selon la revendication 1, où l'électrode externe (18) est formée à partir d'un semi-conducteur ayant des ions accepteurs ou donneurs le dopant.

5. Substrat de réaction biochimique (1) utilisé pour une réaction biochimique, le substrat comprenant :
une zone de réaction (8) pour une réaction biochimique ; et
une électrode (4) pour générer un champ électrique entre elle-même et une électrode externe (18) pour que le champ électrique soit formé à l'intérieur de la zone de réaction ; où :
la réaction biochimique est une réaction d'hybridation d'une chaîne nucléotidique ;
la zone de réaction a un revêtement de surface (5) traité à l'intérieur pour que la chaîne nucléotidique puisse être fixée sur lui ; et
l'électrode (4) est une couche conductrice incluse dans le substrat et formée comme une couche sous-jacente du revêtement de surface.

6. Substrat de réaction biochimique selon la revendication 5, où la couche conductrice est formée dans la zone de réaction comme une couche sous-jacente de la zone de réaction de sorte que le champ électrique généré entre elle-même (4) et l'électrode externe (18) est formé sensiblement perpendiculairement au revêtement de surface.

7. Substrat de réaction biochimique selon la revendication 5, où la couche conductrice forme un champ électrique entre elle-même et une électrode disposée dans une position opposée au revêtement de surface.

8. Substrat de réaction biochimique selon la revendication 5, où le substrat est en forme de disque et a des informations de commande de lecture enregistrées à l'intérieur.

9. Substrat de réaction biochimique selon la revendication 5, où la couche conductrice est transparente à la lumière.

10. Procédé de production d'un substrat d'hybridation (1), le procédé comprenant les étapes de :
formation, sur la surface plate d'un substrat selon la revendication 1, d'une pluralité de puits (8) modifiés chacun en leur fond avec un premier groupe fonctionnel ;
introduction, dans chaque puits, d'une solution contenant une chaîne nucléotidique modifiée à l'une de ses extrémités avec un second groupe fonctionnel qui se combine avec le premier groupe fonctionnel ; et
combinaison du premier groupe fonctionnel avec le second groupe fonctionnel tout en appliquant un champ électrique CA perpendiculaire au substrat plat pour combiner la chaîne nucléotidique avec le fond du puits ; où :
le substrat plat comporte, formée comme une couche sous-jacente du puits, une couche d'électrode (4) formée à partir d'un matériau électriquement conducteur ; et
une électrode externe (18) est prévue à proximité de la surface du substrat pour appliquer une énergie CA entre l'électrode externe et la couche d'électrode afin d'appliquer un champ électrique CA perpendiculairement au substrat plat.

11. Procédé selon la revendication 10, où l'électrode externe est formée à partir d'un semi-conducteur ayant des ions accepteurs ou donneurs le dopant.

12. Procédé d'hybridation comprenant les étapes de :
introduction d'une solution contenant une chaîne nudéotidique utilisée comme échantillon dans un puits (8) formé sur la surface d'un substrat plat selon la revendication 1 et ayant une extrémité d'une chaîne nucléotidique utilisée comme sonde combinée avec son fond ; et
hybridation de la chaîne nucléotidique utilisée comme sonde et de la chaîne nucléotidique utilisée comme échantillon tout en appliquant un champ électrique CA perpendiculairement au substrat plat ; où :
le substrat plat comporte, formée comme une couche sous-jacente du puits, une couche d'électrode formée à partir d'un matériau électriquement conducteur ; et
une électrode externe est disposée à proximité de la surface du substrat pour appliquer une énergie CA entre l'électrode externe et la couche d'électrode pour appliquer un champ électrique CA perpendiculairement au substrat plat.

13. Procédé selon la revendication 12, où l'électrode externe est formée à partir d'un semi-conducteur ayant des ions accepteurs ou donneurs le dopant.
